Europäisches Patentamt

European Patent Office

Office européen des brevets

(19)

(11) Numéro de publication : **0 007 467**
**B1**

## (12) FASCICULE DE BREVET EUROPÉEN

(45) Date de publication du fascicule du brevet :
**11.11.81**

(51) Int. Cl.³ : **A 61 K 39/145**

(21) Numéro de dépôt : **79102214.8**

(22) Date de dépôt : **02.07.79**

(54) **Vaccin antigrippal et procédé pour sa préparation.**

(30) Priorité : **12.07.78 US 923846**

(43) Date de publication de la demande :
**06.02.80 (Bulletin 80/03)**

(45) Mention de la délivrance du brevet :
**11.11.81 Bulletin 81/45**

(84) Etats contractants désignés :
**AT BE CH DE FR GB IT LU NL**

(56) Documents cités :
**BE - A - 843 093**
**FR - A - 2 354 778**

**BULL. ORG. MOND. SANTE ;**
**BULL. WLD. HLTH ORG., 1969, 51, 643-45**
**E.D. KILBOURNE « Future influenza vaccine and the use of genetic recombinants »**

(73) Titulaire : **Smith Kline - RIT Société anonyme dite:**
**rue du Tilleul, 13**
**B-1320 Genval (Rixensart) (BE)**

(72) Inventeur : **Löbmann, Michèle**
**rue d'Angoussart, 165**
**B-1301 Bierges (Wavre) (BE)**
Inventeur : **Florent, Gérard**
**Champ du Cygne, 13**
**B-1320 Genval (Rixensart) (BE)**

(74) Mandataire : **Tasset, Gérard (BE)**
**Smith Kline - RIT 89, rue de l'Institut**
**B-1330 Rixensart (BE)**

EP 0 007 467 B1

Imprimerie Jouve, 17, rue du Louvre, 75001 Paris, France

Vaccin antigrippal et procédé pour sa préparation

La présente invention est relative à un nouveau vaccin vivant antigrippal administrable par voie nasale et à sa préparation.

Jusqu'à présent, on a atténué le virus grippal par différentes techniques, à savoir (a) par recombinaison d'une souche pathogène avec une souche atténuée pour l'homme et antigéniquement différente ; (b) par passages en série du virus à basse température de façon à obtenir un mutant « froid » et, par recombinaison, transfert du caractère « froid » à des souches pathogènes ; (c) par induction de souches thermosensibles (ts) à l'aide d'agents mutagènes et, par recombinaison, transfert du caractère « thermosensible » à des souches pathogènes et (d) par sélection de souches résistantes aux inhibiteurs sériques (R.M. Chanock dans Immunologic and Infectious Reactions in the Lung, édit. Ch.H. Kirkpatrick and H.Y. Reynolds, Marcel Dekker Inc., New York and Basel 1976, p. 390).

On constate que, presque chaque année, le sérotype des souches de virus grippal de type A circulant dans le monde diffère légèrement du sérotype des souches observées jusque là. Cette modification antigénique crée des problèmes particuliers pour l'immunisation contre le virus grippal de type A. En effet, pour obtenir une efficacité maximale, l'antigène vaccinal doit être le plus proche possible de celui du virus dominant et, de ce fait, les vaccins contre la grippe doivent être périodiquement modifiés, de façon à protéger contre ces nouvelles souches.

La présente invention est relative à un vaccin antigrippal contenant une souche de virus grippal de type A (sérotype $H_3N_2$) obtenue par recombinaison de la souche atténuée A/PR/8/34 (Th. Francis, Proc. Soc. Exp. Biol. Med. 32 (1935) p. 1 172-1 175) avec une souche pathogène récemment isolée, à savoir la souche de virus grippal A/Alaska/5/77 et à la préparation de ce vaccin.

Comme il est indiqué ci-avant, la technique de recombinaison d'un variant pathogène avec une souche de virus antigéniquement distincte et connue pour être atténuée pour l'homme était déjà connue. Toutefois, le problème de l'obtention d'un recombinant vaccinal ne réside pas dans la recombinaison mais dans l'isolement d'un recombinant adéquat parmi la nombreuse descendance qui résulte de la recombinaison. Ainsi, l'étude ultérieure de recombinants prometteurs cités par E.D. Kilbourne (Bull. Org. Mond. Santé 1969, 41, p. 643-5) a soit été abandonnée soit révélé que le recombinant n'était pas suffisamment atténué (A.S. Beare and T.S. Hall, The Lancet, Dec 11 1971, p. 1 271-3, Table II). Comme exemples de préparation de souches vaccinales par ce procédé, on peut citer le brevet belge 843 093 et le brevet français 2.354.778 dans lesquels on utilise un marqueur (résistance aux inhibiteurs du sérum) permettant d'éliminer les recombinants qui ne peuvent certainement pas convenir, à cause de leur virulence.

Néanmoins, l'isolement d'une souche vaccinale de la nombreuse descendance virale issue d'une recombinaison notamment au départ d'un nouveau variant reste une opération aléatoire à cause de l'absence de critères in vitro permettant de sélectionner un recombinant particulier qui présenterait toutes les caractéristiques que doit avoir une souche atténuée pour être utilisée comme vaccin.

En recombinant la souche atténuée de virus grippal A/PR/8/34 (qui présente le sérotype $H_0N_1$ et un fort taux de croissance) avec la souche pathogène de virus grippal A/Alaska/5/77 qui présente le sérotype $H_3N_2$ mais constitue un variant des souches $H_3N_2$ précédemment connues, on a pu isoler de la descendance résultant de la recombinaison une nouvelle souche atténuée de virus grippal présentant le sérotype $H_3N_2$. Cette nouvelle souche a été dénommée RIT 4199. Elle est efficace contre la souche A/Alaska/5/77. Son taux d'hybridation RNA/RNA avec la souche A/PR/8/34 a été déterminé par la méthode décrite par G. Florent et al. dans Arch. Virol. 54, 19-28, 1977 ; il s'élève à 67 % (± 3 %). On a trouvé que la souche RIT 4199 est pratiquement non-pathogène et, contrairement aux souches vaccinales précédemment connues, elle convient particulièrement bien pour la production d'un vaccin contre les souches de virus grippal du type A/Texas/1/77, plus particulièrement pour la production d'un vaccin vivant administrable par voie nasale.

La souche de virus grippal A/Alaska/5/77 est une souche sauvage isolée de patients en Alaska. Elle a été obtenue du WHO Collaborating Center for Influenza, Atlanta, Georgia, USA. à son quatrième passage sur œufs SPAFAS (SPAFAS INC., Storrs, Connecticut, USA). Son sérotype est identique à celui de la souche prototype A/Texas/1/77. La souche A/Alaska/5/77 a été déposée le 19 mai 1978 dans la Collection Nationale de Cultures de Microorganismes (C.N.C.M.) de l'Institut Pasteur à Paris sous le n° I-063.

La souche de virus grippal RIT 4199 a été déposée le 19 mai dans la même collection sous le n° I-062.

La présente invention est donc relative à un vaccin vivant contre la grippe comprenant une dose efficace de la souche de virus grippal de type A C.N.C.M. I-062 et un diluant pharmaceutique pour inoculation nasale.

La souche de virus recombinant C.N.C.M. I-062 a été obtenue au départ d'un mélange de quantités sensiblement égales des souches A/PR/8/34 et C.N.C.M. I-063 maintenu au repos pendant quelques heures à 4 °C, en inoculant le mélange dans la cavité allantoïdienne d'œufs embryonnés de poule, en incubant ensuite les œufs inoculés, en récoltant le matériel viral obtenu et en opérant un clonage du matériel viral par passages en dilution limite.

Pour préparer le vaccin suivant l'invention, on cultive la souche de virus grippal recombinant dans des œufs embryonnés et plus particulièrement dans la cavité allantoïdienne d'œufs embryonnés de poule, suivant l'une ou l'autre des techniques connues pour la production de vaccins, de manière à obtenir une quantité importante dudit virus et, si on le désire, on ajoute éventuellement un stabilisateur qui peut être, par exemple, de la peptone ou du saccharose.

Le matériel viral ainsi obtenu est alors réparti dans des flacons contenant des doses unitaires ou multiples de vaccin. Une dose unitaire contient de préférence au moins $10^7 DIO_{50}$ (dose infectieuse dans 50 % des œufs) de virus.

La préparation est de préférence lyophilisée pour assurer sa conversation.

Le vaccin de l'invention est administré par voie nasale, le cas échéant après avoir été réhydraté extemporanément ; à cette fin, on peut utiliser de l'eau purifiée ou tout autre diluant ou composition pharmaceutique connu dans la technique pour la fabrication de préparations nasales sous forme de gouttes ou d'aérosol.

Pour assurer une réponse vaccinale optimale, l'administration du vaccin peut se faire par inoculation de deux doses unitaires successives, la seconde dose étant inoculée une semaine environ après la première.

A plusieurs reprises, dans les exemples qui suivent, il est fait référence à des œufs embryonnés de poule ; dans chaque cas, il s'agit d'œufs provenant d'élevages SPF (specific pathogens free) conformes aux spécifications pour la production et le contrôle de vaccins viraux vivants aviaires, établis en 1976 par le Ministère de l'Agriculture, des Pêcheries et du Ravitaillement de Grande Bretagne.

Les exemples illustrent la présente invention sans en limiter la portée.

Exemple 1

Un échantillon de la souche de virus grippal A/PR/8/34 est reconstitué dans de l'eau distillée et 0,5 ml de cette suspension contenant $10^{8,3} DIO_{50}$ de virus par millilitre est mélangé à 0,5 ml d'une suspension de la souche C.N.C.M. 1-063 dans du liquide allantoïdien d'œufs de poule embryonnés et contenant $10^{8,1} DIO_{50}$ de virus par millilitre et le mélange est maintenu à 4 °C pendant quelques heures. Le mélange est ensuite inoculé dans la cavité allantoïdienne d'œufs embryonnés de poule, lesquels sont incubés à 35 °C pendant 20 heures. On récolte la progéniture de cette culture mixte, on la dilue au dixième (volume/volume) et on la mélange à un même volume de sérum de poule anti A/PR/8/34 dilué au centième (volume/volume) et traité au kaolin. Le mélange est laissé pendant une heure à 37 °C avant d'être inoculé dans la cavité allantoïdienne d'œufs de poule embryonnés où ils sont incubés pendant 24 heures à 35 °C. Un second passage est ensuite effectué dans les mêmes conditions mais pendant 48 heures.

Le virus ainsi obtenu est alors cloné dans la cavité allantoïdienne d'œufs embryonnés de poule par passages en dilution limite, une première fois en présence de sérum de poule anti A/PR/8/34 traité au kaolin et dilué au centième (volume/volume) ; une deuxième fois sans sérum et ensuite deux fois en présence de sérum normal de cobaye.

Une des souches ainsi isolées, a été sélectionnée et caractérisée. Elle a reçu l'appellation RIT 4199 et a été déposée dans la Collection Nationale de Cultures de Microorganismes (C.N.C.M.) de l'Institut Pasteur à Paris sous le n° I-062.

Exemple 2

Caractérisation de la souche de virus grippal C.N.C.M. I-062

La souche C.N.C.M. I-062 a été étudiée au point de vue sérotypie et homologie génétique avec la souche A/PR/8/34 exprimée en pourcentage d'hybridation moléculaire entre le RNA virionique de la souche C.N.C.M. I-062 et le RNA complémentaire de la souche A/PR/8/34 suivant la méthode décrite par G. Florent et al. dans Arch. of Virol. 54, 19-28, 1977.

Les caractéristiques de la souche C.N.C.M. I-062 sont reprises dans le tableau I suivant avec les caractéristiques correspondantes des souches parentales A/PR/8/34 et C.N.C.M. I-063.

TABLEAU I

| Souche | Sérotype | Pourcentage d'hybridation |
|---|---|---|
| A/PR/8/34 | $H_0N_1$ | 100 |
| C.N.C.M. I-063 | $H_3N_2$ | 38 ($\pm$ 3) |
| C.N.C.M. I-062 | $H_3N_2$ | 67 ($\pm$ 3) |

Par identification électrophorétique sur gel de polyacrylamide de l'acide ribonucléique à double hélice (RNA) formé par hybridation, suivant la méthode décrite par A.J. Hay et al. dans Int. Symp. on Influenza Immunization (II), Geneva 1977, Develop. Biol. Standard. 39, 15-23 (S. Karger, Basel 1977), on a déterminé que la souche C.N.C.M. I-062 présente les gènes correspondants aux RNAs 1, 3, 5, 7 et 8 de la souche parente A/PR/8/34 et les gènes correspondants aux RNAs 2, 4 et 6 de la souche parente C.N.C.M. I-063, les RNAs 4 et 6 codant respectivement pour l'hémagglutinine et la neuraminidase.

Exemple 3

Production de vaccin

Comme inoculum pour la production des lots d'ensemencement de vaccin, on utilise le virus grippal, souche C.N.C.M. I-062 récolté au dernier passage de l'exemple 1.

On inocule un échantillon de la souche C.N.C.M. I-062 obtenue à l'exemple 1 dans la cavité allantoïdienne d'œufs embryonnés de

poule qui sont ensuite incubés à 35 °C pendant deux à trois jours.

On récolte et on mélange les liquides allantoïdiens contenant la souche C.N.C.M. I-062, on en teste la stérilité et l'innocuité et on ajoute de la peptone de façon à obtenir une concentration finale de 5 % (volume/volume) en peptone.

On répartit la suspension virale dans des flacons de 3 ml de façon à obtenir des doses unitaires (au moins $10^7 DIO_{50}$) de la souche de virus grippal, on lyophilise et les flacons sont ensuite bouchés hermétiquement.

Le vaccin est reconstitué au moment de l'inoculation par addition de 0,5 ml d'un diluant qui peut être par exemple de l'eau purifiée, du sérum physiologique ou une solution aqueuse de saccharose à 5 % (poids/volume) et on administre dans les narines le vaccin ainsi reconstitué.

Exemple 4

Vaccination avec une dose de vaccin antigrippal atténué, souche C.N.C.M. I-062
Matériel et méthode

On a choisi 14 sujets âgés de 23 à 43 ans (moyenne d'âge : 30 ans) dont le taux d'anticorps déterminé par inhibition de l'hémagglutination était égal ou inférieur à 20, sauf un qui présentait un taux de 40. A chaque sujet on a administré, par instillation de 5 gouttes par narine, une dose de vaccin (souche C.N.C.M. I-062) obtenu à l'exemple 3 et reconstitué dans 0,5 ml d'eau purifiée contenant 5 % (poids/volume) de saccharose. Le vaccin titrait $10^{7.3} DIO_{50}$.

Pour la détermination de la séroconversion (qui correspond soit au passage du taux d'anticorps de <10 à ≤10 soit, lorsque le taux d'anticorps initial est ≥10, à un quadruplement de ce taux d'anticorps, on a prélevé des échantillons sanguins pour la détermination de taux d'anticorps contre la souche C.N.C.M. I-062 respectivement entre deux et trois mois avant vaccination (sauf dans un cas, à savoir le cas 31, pour lequel le prélèvement a été effectué sept mois avant vaccination) et 21 jours après vaccination. Chez six sujets, on a également procédé à des lavages nasaux deux et trois jours après vaccination.
Résultats
  I. Réactions cliniques
  Parmi les 14 vaccinés, 11 n'ont présenté aucun symptôme et trois ont présenté une légère rhinorrhée pendant deux ou trois jours.
  II. Excrétion virale
  Deux et trois jours après vaccination, des lavages nasaux ont été effectués chez six sujets, à savoir les numéros 147, 156, 161, 186 et 190 qui étaient séronégatifs avant vaccination et le n° 31 qui présentait un taux d'anticorps de 40 avant vaccination. Dans aucun cas on n'a détecté une excrétion de virus.
  III. Sérologie
  Le tableau II donne les taux d'anticorps sériques déterminés par inhibition de l'hémagglutination (IH) Contre la souche C.N.C.M. I-062 21 jours après vaccination en face des taux d'anticorps déterminés par inhibition de l'hémagglutination avant vaccination.

TABLEAU II
Taux d'anticorps sériques (exprimés en unités IH)

| Sujets | Unités IH | |
|---|---|---|
| | avant vaccination | après vaccination |
| 159 | < 10 | < 10 |
| 148 | < 10 | ≤ 10 |
| 156 | < 10 | 10 |
| 172 | < 10 | 10 |
| 190 | < 10 | 10 |
| 189 | < 10 | 10/20 |
| 147 | < 10 | 20 |
| 171 | < 10 | 20 |
| 186 | < 10 | 20 |
| 161 | < 10 | 40/80 |
| 220 | < 10 | 80 |
| 200 | 10 | 320 |
| 157 | 20 | 40/80 |
| 31 | 40 | 160 |

De l'examen du tableau II, il résulte que, parmi les sujets présentant un taux d'anticorps IH≤20 avant vaccination, 11 sur 13 (soit 85 %) ont séroconverti et que le sujet qui présentait un taux d'anticorps IH de 40 avant vaccination a également séroconverti. Sur l'ensemble des 14 sujets, douze (c'est-à-dire 86 %) ont séroconverti.

Le titre géométrique moyen du sous-groupe qui présentait un titre égal ou inférieur à 20 avant vaccination s'est élevé de 6 à 20. On peut donc conclure que le vaccin n'a causé que des réactions insignifiantes, que le virus n'est pas excrété et provoque chez la plupart des sujets une réponse satisfaisante en anticorps sériques déterminés par inhibition de l'hémagglutination IH.

Exemple 5

Vaccination avec deux doses successives de vaccin antigrippal atténué, souche C.N.C.M. I-062
Matériel et méthode

A 14 sujets âgés de 19 à 42 ans (moyenne d'âge : 25 ans) on a administré à sept jours d'intervalle deux doses de vaccin (souche C.N.C.M. I-062) obtenu à l'exemple 3 et reconstitué extemporanément dans 0,5 ml d'une solution de saccharose à 5 % (poids/volume) dans de l'eau purifiée. Le vaccin titrait $10^{7.3} DIO_{50}$ et a été administré par instillation de cinq gouttes par narine. Les taux d'anticorps déterminés par inhibition de l'hémagglutination IH sont indiqués au tableau III. Les échantillons sanguins pour la sélection des sujets avaient été prélevés trois jours avant la première administration de vaccin. Le taux d'anticorps des sujets vaccinés a été déterminé sur la base d'échantillons sanguins prélevés 21 jours après la première administration.
Résultats
  1. Réactions cliniques
  Les vaccinés ont été surveillés au point de vue de l'apparition éventuelle de symptômes tels que rhinorrhée, enrouement, nez bouché, mal de gorge, toux, nature des expectorations et mal de tête. Parmi eux, six (les numéros 167, 236, 240, 241, 242 et 245) n'ont présenté aucun de ces symptômes, un (le n° 243) a présenté pendant deux jours une légère rhinorrhée ; un (le n° 234) a présenté une légère toux mais dès le jour de la vaccination ; un (le n° 238) a présenté une légère rhinorrhée les troisième et quatrième jours après vaccination, un nez modérément bouché les sixième et septième jours et une toux modérée les troisième et septième jours. Le sujet n° 233 a signalé un mal de tête modéré pendant sept jours à partir du jour de vaccination. Le sujet n° 235 a signalé un mal de tête léger à modéré entre le premier et sixième jour. Chez un sujet (le n° 244), on a noté quatre élévations de température (au moins 37,5 °C) dans la soirée du troisième au sixième jour, un nez modérément bouché du quatrième au sixième jour, une sérieuse rhinorrhée du deuxième au troisième jour, un mal de gorge modéré à sérieux du deuxième au septième jour et une toux sévère du quatrième au septième jour. Le même sujet s'est également plaint d'un mal de tête moyen à sérieux du deuxième au sixième jour et il n'a pas séroconverti après vaccination. Pour les sujets numéros 237 et 239, on n'a pas obtenu de fiche de contrôle de symptômes mais, étant donné que ces sujets n'ont pas repris contact avec l'expérimentateur, on peut supposer qu'ils n'ont pas eu d'effets secondaires importants. Les sujets qui n'ont signalé qu'un mal de tête peuvent probablement être exclus du groupe ayant présenté des réactions liées à la vaccination.

2. Sérologie

Le tableau III suivant donne les taux d'anticorps sériques déterminés par inhibition de l'hémagglutination (IH) contre la souche C.N.C.M. I-062, 21 jours après vaccination en face des taux d'anticorps sériques déterminés par inhibition de l'hémagglutination avant vaccination.

TABLEAU III
Taux d'anticorps sériques (exprimés en unités IH)
contre la souche C.N.C.M. I-062

| Sujets | Unités IH | |
|---|---|---|
| | avant vaccination | après vaccination |
| 244 | < 10 | < 10 |
| 243 | < 10 | 10 |
| 240 | < 10 | 10 |
| 235 | < 10 | 10 |
| 233 | < 10 | 20 |
| 237 | < 10 | 40 |
| 239 | < 10 | 40 |
| 241 | < 10 | 40 |
| 242 | < 10 | 320 |
| 238 | < 10 | ≥ 640 |
| 236 | < 10 | 160 |
| 167 | 10 | 40 |
| 234 | 10 | 320 |
| 245 | 80 | 320 |

De l'examen de ce tableau III, il résulte que, parmi les 13 sujets présentant un taux d'anticorps égal ou inférieur à dix avant vaccination, 12 (c'est-à-dire 92,3 %) ont séroconverti et le sujet (n° 245) qui présentait un taux d'anticorps de 80 avant vaccination a également séroconverti.

Pour le sous-groupe qui présentait avant vaccination un taux d'anticorps IH égal ou inférieur à dix, le titre géométrique moyen est passé de six avant vaccination à ≥ 44 trois semaines après l'administration de la première dose de vaccin et, pour l'ensemble des sujets, le titre est passé de 7 à ≥ 51.

Il apparaît également de l'expérimentation que le vaccin ne produit que des réactions bénignes qui sont cliniquement acceptables.

**Revendications** pour les États contractants : BE, CH, DE, FR, GB, IT, LU et NL

1. Vaccin vivant antigrippal caractérisé en ce qu'il contient la souche de virus grippal déposée dans la Collection Nationale des Cultures de Microorganismes de l'Institut Pasteur à Paris sous le numéro : C.N.C.M. I-062 et un diluant pharmaceutique pour administration nasale.

2. Vaccin vivant antigrippal suivant la revendication 1, caractérisé en ce que la dose unitaire de virus est au moins $10^7 DIO_{50}$.

3. Vaccin suivant la revendication 1 ou la revendication 2, caractérisé en ce qu'il est présenté sous forme lyophilisée.

4. Procédé pour la préparation d'un vaccin vivant antigrippal administrable par voie nasale, caractérisé en ce qu'on cultive la souche de virus grippal déposée dans la Collection Nationale des Cultures de Microorganismes de l'Institut Pasteur à Paris sous le numéro C.N.C.M. I-062 dans la cavité allantoïdienne d'œufs de poule embryonnés pendant une durée suffisante pour permettre la production d'une importante quantité dudit virus, qu'on récolte le matériel viral produit et que, si on le désire, on y ajoute un stabilisant et on lyophilise le mélange.

**Revendication** pour l'État contractant : AT.

1. Procédé pour la préparation d'un vaccin vivant antigrippal administrable par voie nasale, caractérisé en ce qu'on cultive la souche de virus grippal déposée dans la Collection Nationale des Cultures de Microorganismes de l'Institut Pasteur à Paris sous le numéro C.N.C.M. I-062 dans la cavité allantoïdienne d'œufs de poule embryonnés pendant une durée suffisante pour permettre la production d'une importante quantité dudit virus, qu'on récolte le matériel viral produit et que, si on le désire, on y ajoute un stabilisant et on lyophilise le mélange.

**Claims** for the Contracting States : BE, CH, DE, FR, GB, IT, LU and NL.

1. Live influenza virus vaccine comprising the influenza virus C.N.C.M. I-062 strain and a pharmaceutical diluent for nasal administration.

2. Live influenza virus vaccine according to claim 1 wherein the virus dosage unit is at least $10^7 EID_{50}$.

3. Vaccine according to claim 1 or claim 2 in freeze-dried form.

4. Process for the preparation of a live intranasal influenza virus vaccine comprising allowing the influenza virus strain deposited with the National Collection of Culture of Microorganisms under C.N.C.M. number I-062 to grow in the allantoïc cavity of fertile hen eggs for a period of time sufficient to permit production of a large amount of said virus, harvesting the resulting virus material and, if desired, adding thereto a stabilizer and freeze-drying the mixture.

Claim for the Contracting State : AT.

1. Process for the preparation of a live intranasal influenza virus vaccine comprising allowing the influenza virus strain deposited with the National Collection of Culture of Microorganisms under C.N.C.M. number I-062 to grow in the allantoïc cavity of fertile hen eggs for a period of time sufficient to permit production of a large amount of said virus, harvesting the resulting virus material and, if desired, adding thereto a stabilizer and freeze-drying the mixture.

Ansprüche für die Vertragsstaaten : BE, CH, DE, FR, GB, IT, LU und NL.

1. Influenza-Lebendimpfstoff, dadurch gekennzeichnet, daß er den Influenzavirus-Stamm C.N.C.M. I-062 und ein pharmazeutisches Ver-dünnungsmitel zur nasalen Verabreichung enthält.

2. Influenza-Lebendimpfstoff nach Anspruch 1, dadurch gekennzeichnet, daß die Einzeldosis mindestens $10^7 DIO_{50}$ des Virus enthält.

3. Impfstoff nach Anspruch 1 oder 2, dadurch gekennzeichnet, daß er in lyophilisierter Form vorliegt.

4. Verfahren zur Herstellung eines auf nasalem Weg verabreichbaren Influenza-Lebendimpfstoffs, dadurch gekennzeichnet, daß man den Influenzavirus-Stamm, der in der Collection Nationale des Cultures de Microorganismes des Institut Pasteur in Paris unter der Nummer C.N.C.M. I-062 hinterlegt ist, in der Allantoishöhle bebrüteter Hühnereier ausreichend lange kultiviert, um die Erzeugung einer wesentlichen Menge des Virus zu ermöglichen, das erzeugte Virusmaterial erntet und gewünschtenfalls mit einem Stabilisator versetzt und das Gemisch lyophilisiert.

Anspruch für den Vertragsstaat : AT.

1. Verfahren zur Herstellung eines auf nasalem Weg verabreichbaren Influenza-Lebendimpfstoffs, dadurch gekennzeichnet, daß man den Influenzavirus-Stamm, der in der Collection Nationale des Cultures de Microorganismes des Instituts Pasteur in Paris unter der Nummer C.N.C.M. I-062 hinterlegt ist, in der Allantoishöhle bebrüteter Hühnereier ausreichend lange kultiviert, um die Erzeugung einer wesentlichen Menge des Virus zu ermöglichen, das erzeugte Virusmaterial erntet und gewünschtenfalls mit einem Stabilisator versetzt und das Gemisch lyophilisiert.